# EUROPEAN PATENT SPECIFICATION

(11) **EP 0 783 583 B1**
(45) Date of publication and mention of the grant of the patent: **07.06.2006**
(21) Application number: 95934153.8
(22) Date of filing: 09.10.1995
(51) Int. Cl.: C12N 15/54, C12Q 1/68, C07K 16/40, C12N 9/12

(54) **CYTOPLASMIC TYROSINE KINASE**
CYTOPLASMISCHE TYROSIN KINASE
TYROSINE KINASE CYTOPLASMIQUE

(30) Priority: 07.10.1994 US 320432
(43) Date of publication of application: 16.07.1997
(73) Proprietor: Licentia Ltd., 00290 Helsinki (FI)
(72) Inventor: ALITALO, Kari, 02100 Espoo (FI)
(74) Representative: Karvinen, Leena Maria
(86) International application number: PCT/FI1995/000555
(87) International publication number: WO 1996/011275

(56) References cited:
- PROCEEDINGS OF THE NATIONAL ACADEMY OF SCIENCES OF USA, vol. 86, 1989 WASHINGTON US, pages 1603-1607, A. WILKS 'Two putative protein-tyrosine kinases identified by application of the polymerase chain reaction'
- ONCOGENE (1994), 9(4), 1155-61 CODEN: ONCNES;ISSN: 0950-9232, April 1994 SAKANO, SEIJI ET AL 'Molecular cloning of a novel non - receptor tyrosine kinase, HYL ( hematopoietic consensus tyrosine-lacking kinase)'
- NATURE, vol. 361, 21 January 1993 pages 226-233, D. VETRIE ET AL 'The gene involved in X-linked agammaglobulinaemia is a member of the src family of protein-tyrosine kinases' cited in the application
- ONCOGENE (1994), 9(12), 3683-8 CODEN: ONCNES;ISSN: 0950-9232, December 1994 TAMAGNONE, LUCA ET AL 'BMX, a novel nonreceptor tyrosine kinase gene of the BTK ITK TEC TXK family located in chromosome Xp22.2'

### SEQUENCE LISTING

(1) GENERAL INFORMATION:
   (i) APPLICANT: Helsinki University Licensing Ltd Oy
   (ii) TITLE OF INVENTION: Cytoplasmic Tyrosine Kinase
   (iii) NUMBER OF SEQUENCES: 8
   (iv) CORRESPONDENCE ADDRESS:
      (A) ADDRESSEE: OY JALO ANT-WUORINEN AB
      (B) STREET: Iso Roobertinkatu 4-6 A
      (C) CITY: Helsinki
      (E) COUNTRY: Finland
      (F) ZIP: FIN-00120
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Karvinen, Leena
      (C) REFERENCE/DOCKET NUMBER: 28218
   (ix) TELECOMMUNICATION INFORMATION:
      (A) TELEPHONE: +358 0 648606
      (B) TELEFAX: +358 0 640575
      (C) TELEX: 123505 JALO FI
(2) INFORMATION FOR SEQ ID NO:1:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
      GGTCTAGAAR AARTTYGTSC ACMGRGAC 28
(2) INFORMATION FOR SEQ ID NO:2:
   (i) SEQUENCE CHARACTERISTICS:
      (A) LENGTH: 27 base pairs
      (B) TYPE: nucleic acid
      (C) STRANDEDNESS: single
      (D) TOPOLOGY: linea:
   (ii) MOLECULE TYPE: DNA (genomic)
   (xi) SEQUENCE DESCRIPTION: SEQ ID NO:2:
      GCTCTAGARG GCCATCCAYT TVACHGG 27
(2) INFORMATION FOR SEQ ID NO:3:
   (i) SEQUENCE CHARACTERISTICS:
      (A) LENGTH: 675 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:3:
   Met Asp Thr Lys Ser Ile Leu Glu Glu Leu Leu Leu Lys Arg Ser Gln
   1               5                   10                  15
   Gln Lys Lys Lys Met Ser Pro Asn Asn Tyr Lys Glu Arg Leu Phe Val
               20                  25                  30
   Leu Thr Lys Thr Asn Leu Ser Tyr Tyr Glu Tyr Asp Lys Met Lys Arg
           35                  40                  45
   Gly Ser Arg Lys Gly Ser Ile Glu Ile Lys Lys Ile Arg Cys Val Glu
       50                  55                  60
   Lys Val Asn Leu Glu Glu Gln Thr Pro Val Glu Arg Gln Tyr Pro Phe
   65                  70                  75                  80
   Gln Ile Val Tyr Lys Asp Gly Leu Leu Tyr Val Tyr Ala Ser Asn Glu
                   85                  90                  95
   Glu Ser Arg Ser Gln Trp Leu Lys Ala Leu Gln Lys Glu Ile Arg Gly
               100                 105                 110
   Asn Pro His Leu Leu Val Lys Tyr His Ser Gly Phe Phe Val Asp Gly
           115                 120                 125
   Lys Phe Leu Cys Cys Gln Gln Ser Cys Lys Ala Ala Pro Gly Cys Thr
       130                 135                 140
   Leu Trp Glu Ala Tyr Ala Asn Leu His Thr Ala Val Asn Glu Glu Lys
   145                 150                 155                 160
   His Arg Val Pro Thr Phe Pro Asp Arg Val Leu Lys Ile Pro Arg Ala
                   165                 170                 175
   Val Pro Val Leu Lys Met Asp Ala Pro Ser Ser Ser Thr Thr Leu Ala
               180                 185                 190
   Gln Tyr Asp Asn Glu Ser Lys Lys Asn Tyr Gly Ser Gln Pro Pro Ser
           195                 200                 205
   Ser Ser Thr Ser Leu Ala Gln Tyr Asp Ser Asn Ser Lys Lys Ile Tyr
       210                 215                 220
   Gly Ser Gln Pro Asn Phe Asn Met Gln Tyr Ile Pro Arg Glu Asp Phe
   225                 230                 235                 240
   Pro Asp Trp Trp Gln Val Arg Lys Leu Lys Ser Ser Ser Ser Ser Glu
                   245                 250                 255
   Asp Val Ala Ser Ser Asn Gln Lys Glu Arg Asn Val Asn His Thr Thr
               260                 265                 270
   Ser Lys Ile Ser Trp Glu Phe Pro Glu Ser Ser Ser Ser Glu Glu Glu
           275                 280                 285
   Glu Asn Leu Asp Asp Tyr Asp Trp Phe Ala Gly Asn Ile Ser Arg Ser
       290                 295                 300
   Gln Ser Glu Gln Leu Leu Arg Gln Lys Gly Lys Glu Gly Ala Phe Met
   305                 310                 315                 320
   Val Arg Asn Ser Ser Gln Val Gly Met Tyr Thr Val Ser Leu Phe Ser
                   325                 330                 335
   Lys Ala Val Asn Asp Lys Lys Gly Thr Val Lys His Tyr His Val His
               340                 345                 350
   Thr Asn Ala Glu Asn Lys Leu Tyr Leu Ala Glu Asn Tyr Cys Phe Asp
           355                 360                 365
   Ser Ile Pro Lys Leu Ile His Tyr His Gln His Asn Ser Ala Gly Met
       370                 375                 380
   Ile Thr Arg Leu Arg His Pro Val Ser Thr Lys Ala Asn Lys Val Pro
   385                 390                 395                 400
   Asp Ser Val Ser Leu Gly Asn Gly Ile Trp Glu Leu Lys Arg Glu Glu
                   405                 410                 415
   Ile Thr Leu Leu Lys Glu Leu Gly Ser Gly Gln Phe Gly Val Val Gln
               420                 425                 430
   Leu Gly Lys Trp Lys Gly Gln Tyr Asp Val Ala Val Lys Met Ile Lys
           435                 440                 445
   Glu Gly Ser Met Ser Glu Asp Glu Phe Phe Gln Glu Ala Gln Thr Met
       450                 455                 460
   Met Lys Leu Ser His Pro Lys Leu Val Lys Phe Tyr Gly Val Cys Ser
   465                 470                 475                 480
   Lys Glu Tyr Pro Ile Tyr Ile Val Thr Glu Tyr Ile Ser Asn Gly Cys
                   485                 490                 495
   Leu Leu Asn Tyr Leu Arg Ser His Gly Lys Gly Leu Glu Pro Ser Gln
               500                 505                 510
   Leu Leu Glu Met Cys Tyr Asp Val Cys Glu Gly Met Ala Phe Leu Glu
           515                 520                 525
   Ser His Gln Phe Ile His Arg Asp Leu Ala Ala Arg Asn Cys Leu Val
       530                 535                 540
   Asp Arg Asp Leu Cys Val Lys Val Ser Asp Phe Gly Met Thr Arg Tyr
   545                 550                 555                 560
   Val Leu Asp Asp Gln Tyr Val Ser Ser Val Gly Thr Lys Phe Pro Val
                   565                 570                 575
   Lys Trp Ser Ala Pro Glu Val Phe His Tyr Phe Lys Tyr Ser Ser Lys
               580                 585                 590
   Ser Asp Val Trp Ala Phe Gly Ile Leu Met Trp Glu Val Phe Ser Leu
           595                 600                 605
   Gly Lys Gln Pro Tyr Asp Leu Tyr Asp Asn Ser Gln Val Val Leu Lys
       610                 615                 620
   Val Ser Gln Gly His Arg Leu Tyr Arg Pro His Leu Ala Ser Asp Thr
   625                 630                 635                 640
   Ile Tyr Gln Ile Met Tyr Ser Cys Trp His Glu Leu Pro Glu Lys Arg
                   645                 650                 655
   Pro Thr Phe Gln Gln Leu Leu Ser Ser Ile Glu Pro Leu Arg Glu Lys
               660                 665                 670
   Asp Lys His
           675
(2) INFORMATION FOR SEQ ID NO:4:
   (i) SEQUENCE CHARACTERISTICS:
      (A) LENGTH: 659 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 620 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:5:
   Met Asn Asn Phe Ile Leu Leu Glu Glu Gln Leu Ile Lys Lys Ser Gln
   1               5                   10                  15
   Gln Lys Arg Arg Thr Ser Pro Ser Asn Phe Lys Val Arg Phe Phe Val
               20                  25                  30
   Leu Thr Lys Ala Ser Leu Ala Tyr Phe Glu Asp Arg His Gly Lys Lys
           35                  40                  45
   Arg Thr Leu Lys Gly Ser Ile Glu Leu Ser Arg Ile Lys Cys Val Glu
       50                  55                  60
   Ile Val Lys Ser Asp Ile Ser Ile Pro Cys His Tyr Lys Tyr Pro Phe
   65                  70                  75                  80
   Gln Val Val His Asp Asn Tyr Leu Leu Tyr Val Phe Ala Pro Asp Arg
                   85                  90                  95
   Glu Ser Arg Gln Arg Trp Val Leu Ala Leu Lys Glu Glu Thr Arg Asn
               100                 105                 110
   Asn Asn Ser Leu Val Pro Lys Tyr His Pro Asn Phe Trp Met Asp Gly
           115                 120                 125
   Lys Trp Arg Cys Cys Ser Gln Leu Glu Lys Leu Ala Thr Gly Cys Ala
       130                 135                 140
   Gln Tyr Asp Pro Thr Lys Asn Ala Ser Lys Lys Pro Leu Pro Pro Thr
   145 150 155 160
   Pro Glu Asp Asn Arg Arg Pro Leu Trp Glu Pro Glu Glu Thr Val Val
   165 170 175
   Ile Ala Leu Tyr Asp Tyr Gln Thr Asn Asp Pro Gln Glu Leu Ala Leu
   180 185 190
   Arg Arg Asn Glu Glu Tyr Cys Leu Leu Asp Ser Ser Glu Ile His Trp
   195 200 205
   Trp Arg Val Gln Asp Arg Asn Gly His Glu Gly Tyr Val Pro Ser Ser
   210 215 220
   Tyr Leu Val Glu Lys Ser Pro Asn Asn Leu Glu Thr Tyr Glu Trp Tyr
   225 230 235 240
   Asn Lys Ser Ile Ser Arg Asp Lys Ala Glu Lys Leu Leu Leu Asp Thr
   245 250 255
   Gly Lys Glu Gly Ala Phe Met Val Arg Asp Ser Arg Thr Ala Gly Thr
   260 265 270
   Tyr Thr Val Ser Val Phe Thr Lys Ala Val Val Ser Glu Asn Asn Pro
   275 280 285
   Cys Ile Lys His Tyr His Ile Lys Glu Thr Asn Asp Asn Pro Lys Arg
   290 295 300
   Tyr Tyr Val Ala Glu Lys Tyr Val Phe Asp Ser Ile Pro Leu Leu Ile
   305 310 315 320
   Asn Tyr His Gln His Asn Gly Gly Gly Leu Val Thr Arg Leu Arg Tyr
   325 330 335
   Pro Val Cys Phe Gly Arg Gln Lys Ala Pro Val Thr Ala Gly Leu Arg
   340 345 350
   Tyr Gly Lys Trp Val Ile Asp Pro Ser Glu Leu Thr Phe Val Gln Glu
   355 360 365
   Ile Gly Ser Gly Gln Phe Gly Leu Val His Leu Gly Tyr Trp Leu Asn
   370 375 380
   Lys Asp Lys Val Ala Ile Lys Thr Ile Arg Glu Gly Ala Met Ser Glu
   385 390 395 400
   Glu Asp Phe Ile Glu Glu Ala Glu Val Met Met Lys Leu Ser His Pro
   405 410 415
   Lys Leu Val Gln Leu Tyr Gly Val Cys Leu Glu Gln Ala Pro Ile Cys
   420 425 430
   Leu Val Phe Glu Phe Met Glu His Gly Cys Leu Ser Asp Tyr Leu Arg
   435 440 445
   Thr Gln Arg Gly Leu Phe Ala Ala Glu Thr Leu Leu Gly Met Cys Leu
   450 455 460
   Asp Val Cys Glu Gly Met Ala Tyr Leu Glu Glu Ala Cys Val Ile His
   465 470 475 480
   Arg Asp Leu Ala Ala Arg Asn Cys Leu Val Gly Glu Asn Gln Val Ile
   485 490 495
   Lys Val Ser Asp Phe Gly Met Thr Arg Phe Val Leu Asp Asp Gln Tyr
               500                 505                 510
   Thr Ser Ser Thr Gly Thr Lys Phe Pro Val Lys Trp Ala Ser Pro Glu
           515                 520                 525
   Val Phe Ser Phe Ser Arg Tyr Ser Ser Lys Ser Asp Val Trp Ser Phe
       530                 535                 540
   Gly Val Leu Met Trp Glu Val Phe Ser Glu Gly Lys Ile Pro Tyr Glu
   545                 550                 555                 560
   Asn Arg Ser Asn Ser Glu Val Val Glu Asp Ile Ser Thr Gly Phe Arg
                   565                 570                 575
   Leu Tyr Lys Pro Arg Leu Ala Ser Thr His Val Tyr Gln Ile Met Asn
               580                 585                 590
   His Cys Trp Lys Glu Arg Pro Glu Asp Arg Pro Ala Phe Ser Arg Leu
           595                 600                 605
   Leu Arg Gln Leu Ala Glu Ile Ala Glu Ser Gly Leu
       610                 615                 620
(2) INFORMATION FOR SEQ ID NO:6:
   (i) SEQUENCE CHARACTERISTICS:
      (A) LENGTH: 630 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:6:
   Met Asn Phe Asn Thr Ile Leu Glu Glu Ile Leu Ile Lys Arg Ser Gln
   1               5                   10                  15
   Gln Lys Lys Lys Thr Ser Leu Leu Asn Tyr Lys Glu Arg Leu Cys Val
               20                  25                  30
   Leu Pro Lys Ser Val Leu Ser Tyr Tyr Glu Gly Arg Ala Glu Lys Lys
           35                  40                  45
   Tyr Arg Lys Gly Val Ile Asp Ile Ser Lys Ile Lys Cys Val Glu Ile
       50                  55                  60
   Val Lys Asn Asp Asp Gly Val Ile Pro Cys Gln Asn Lys Phe Pro Phe
   65                  70                  75                  80
   Gln Val Val His Asp Ala Asn Thr Leu Tyr Ile Phe Ala Pro Ser Pro
                   85                  90                  95
   Gln Ser Arg Asp Arg Trp Val Lys Lys Leu Lys Glu Glu Ile Lys Asn
               100                 105                 110
   Asn Asn Asn Ile Met Ile Lys Tyr His Pro Lys Phe Trp Ala Asp Gly
           115                 120                 125
   Ser Tyr Gln Cys Cys Arg Gln Thr Glu Lys Leu Ala Pro Gly Cys Glu
       130                 135                 140
   Lys Tyr Asn Leu Phe Glu Ser Ser Ile Arg Lys Thr Leu Pro Pro Ala
   145                 150                 155                 160
   Pro Glu Ile Lys Lys Arg Arg Pro Pro Pro Pro Ile Pro Pro Glu Lys
                   165                 170                 175
   Lys Asn Thr Glu Glu Ile Val Val Ala Met Tyr Asp Phe Gln Ala Thr
               180                 185                 190
   Glu Ala His Asp Leu Arg Leu Glu Arg Gly Gln Glu Tyr Ile Ile Leu
           195                 200                 205
   Glu Lys Asn Asp Leu His Trp Trp Arg Ala Arg Asp Lys Tyr Gly Ser
       210                 215                 220
   Glu Gly Tyr Ile Pro Ser Asn Tyr Val Thr Gly Lys Lys Ser Asn Asn
   225                 230                 235                 240
   Leu Asp Gln Tyr Glu Trp Tyr Cys Arg Asn Thr Asn Arg Ser Lys Ala
                   245                 250                 255
   Glu Gln Leu Leu Arg Thr Glu Asp Lys Glu Gly Gly Phe Met Val Arg
               260                 265                 270
   Asp Ser Ser Gln Pro Gly Leu Tyr Thr Val Ser Leu Tyr Thr Lys Phe
           275                 280                 285
   Gly Gly Glu Gly Ser Ser Gly Phe Arg His Tyr His Ile Lys Glu Thr
       290                 295                 300
   Ala Thr Ser Pro Lys Lys Tyr Tyr Leu Ala Glu Lys His Ala Phe Gly
   305                 310                 315                 320
   Ser Ile Pro Glu Ile Ile Glu Tyr His Lys His Asn Ala Ala Gly Leu
                   325                 330                 335
   Val Thr Arg Leu Arg Tyr Pro Val Ser Thr Lys Gly Lys Asn Ala Pro
               340                 345                 350
   Thr Thr Ala Gly Phe Ser Tyr Asp Lys Trp Glu Ile Asn Pro Ser Glu
           355                 360                 365
   Leu Thr Phe Met Arg Glu Leu Gly Ser Gly Leu Phe Gly Val Val Arg
       370                 375                 380
   Leu Gly Lys Trp Arg Ala Gln Tyr Lys Val Ala Ile Lys Ala Ile Arg
   385                 390                 395                 400
   Glu Gly Ala Met Cys Glu Glu Asp Phe Ile Glu Glu Ala Lys Val Met
                   405                 410                 415
   Met Lys Leu Thr His Pro Lys Leu Val Gln Leu Tyr Gly Val Cys Thr
               420                 425                 430
   Gln Gln Lys Pro Ile Tyr Ile Val Thr Glu Phe Met Glu Arg Gly Cys
           435                 440                 445
   Leu Leu Asn Phe Leu Arg Gln Arg Gln Gly His Phe Ser Arg Asp Met
       450                 455                 460
   Leu Leu Ser Met Cys Gln Asp Val Cys Glu Gly Met Glu Tyr Leu Glu
   465                 470                 475                 480
   Arg Asn Ser Phe Ile His Arg Asp Leu Ala Ala Arg Asn Cys Leu Val
                   485                 490                 495
   Asn Glu Ala Gly Val Val Lys Val Ser Asp Phe Gly Met Ala Arg Tyr
   500 505 510
   Val Leu Asp Asp Gln Tyr Thr Ser Ser Ser Gly Ala Lys Phe Pro Val
   515 520 525
   Lys Trp Cys Pro Pro Glu Val Phe Asn Tyr Ser Arg Gly Ser Ser Lys
   530 535 540
   Ser Asp Val Trp Ser Phe Gly Val Leu Met Trp Glu Ile Phe Thr Glu
   545 550 555 560
   Gly Arg Met Pro Phe Glu Lys Asn Thr Asn Tyr Glu Val Val Thr Met
   565 570 575
   Val Thr Arg Gly His Arg Leu His Arg Pro Lys Leu Ala Thr Lys Tyr
   580 585 590
   Leu Tyr Glu Val Met Leu Arg Cys Trp Gln Glu Arg Pro Glu Gly Arg
   595 600 605
   Pro Ser Phe Glu Asp Leu Leu Arg Thr Ile Asp Glu Leu Val Glu Cys
   610 615 620
   Glu Glu Thr Phe Gly Arg
   625 630
(2) INFORMATION FOR SEQ ID NO:7:
   (i) SEQUENCE CHARACTERISTICS:
      (A) LENGTH: 85 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:7:
   Val Ile Lys Glu Gly Leu Lys Lys Trp Lys Arg Phe Val Leu Leu Ser
   1 5 10 15
   Tyr Tyr Lys Gly Leu Ile Asp Leu Ile Ile Val Glu Phe Ile Val Leu
   20 25 30
   Ile Leu Ala Glu Glu Glu Arg Trp Val Ala Leu Ile Ala Leu Tyr Asp
   35 40 45
   Tyr Asp Leu Leu Gly Asp Ile Leu Trp Trp Gly Pro Tyr Val Trp Ile
   50 55 60
   Ser Arg Ala Leu Leu Gly Phe Leu Val Arg Gly Tyr Ser Val His Tyr
   65 70 75 80
   Phe Leu Ile Pro Val
   85
(2) INFORMATION FOR SEQ, ID NO:8:
   (i) SEQUENCE CHARACTERISTICS
      (A) LENGTH: 441 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:8:

## Description

### FIELD OF THE INVENTION

The present invention generally relates to a novel cytoplasmic tyrosine kinase, the gene encoding it, and its use in diagnostic and therapeutic procedures.

### BACKGROUND OF THE INVENTION

Cellular processes involved in the maintenance, differentiation, and repair of cells and tissues are regulated, in part, by intercellular and intracellular signals which are controlled by the binding of growth factors and other ligands to their receptors. One important mode of signalling used by cells to regulate gene expression and activation or deactivation of biochemical pathways is tyrosine phosphorylation. Numerous tyrosine kinases are known and they usually exist as transmembrane receptors for polypeptide growth factors, such as epidermal growth factor, insulin, insulin-like growth factor I,platelet-derived growth factors, and fibroblast growth factors. *See,* generally, Ullrich, *et al., Cell, 61*:243-254 (1990); and Heldin, *et al*., *Cell Regulation, 1*:555-556 (1990). Of interest to the present invention are several receptor tyrosine kinases which recognize hematopoietic growth factors as their ligands. These include the c-fms receptor tyrosine kinase which is the receptor for colony-stimulating factor 1, Sherr, *et al., Cell 41:* 665-676 (1985), and c-kit, a primitive and less well-characterized hematopoietic growth factor reported in Huang, *et al., Cell 63:* 225-233 (1990).

Tyrosine kinases are generally divided into families, subfamilies, and classes based upon structural characteristics. In general, two broad classifications exist and those include membrane-bound tyrosine receptor kinases and nonreceptor tyrosine kinases, which include cytoplasmic and nuclear tyrosine kinases. An example of tyrosine receptor kinases is the family of epidermal growth factor receptor kinases which form a subfamily of transmembrane receptor kinases with extracellular domains. That subfamily, along with others, such as insulin receptor kinases, contain homologous cysteine-rich repeats in their extracellular domains. *See* Hirai, *et al., Science, 238*: 1717-1720 (1987). Other membrane-bound receptor tyrosine kinases contain extracellular folds which are characteristic of the immunoglobulin superfamily.

The nonreceptor tyrosine kinases are often referred to as a single group comprising Src-like kinases, but it is now recognized that the nonreceptor tyrosine kinases may be divided into several families. Bolen, *Oncogene, 8*: 2025-2031 (1993); Wang, *TIBS 19,* 373-376, 1994.

For example, a newly-identified non-receptor tyrosine kinase family includes three independently-cloned genes, *TEC, ITK* (also known as *TSK* or *EMT),* and *BTK* (formerly known as *ATK,* or EMB). Proteins encoded by those genes are generally homologous to the *Drosophila melanogaster* Src28C tyrosine kinase. Such peptides generally contain SH3 and SH2 domains upstream of the tyrosine kinase domain. However, peptides encoded by *TEC*/*ITK*/*BTR* also typically contain a long N-terminal region which does not have a consensus myristylation residue which is generally conserved in Src-like kinases. Instead, the N-terminal regions of proteins of the *TEC*/*ITK*/*BTK* family contain a pleckstrin homology (PH) domain which is described in Musacchio, *et al*., *TIBS, 18*: 343-348 (1993). Finally, the *TEC*/*ITK*/*BTK* family generally consists of peptides which have a short C-terminus, lacking the regulatory tyrosine phosphorylation site found in most Src-like kinases.

The core of the pleckstrin domain is an antiparallel beta-sheet consisting of seven strands. The C-terminus is folded into a long alpha-helix. The domain is electrostatically polarized and contains a pocket which may be involved in binding to a ligand, such as a peptide or a small protein. This core structure is conserved in all PH domains, which, however, have large variations in the loops surrounding the putative binding pocket. The functions of pleckstrin domains are still unknown, although they have been shown to bind to the β and γ subunits of complex G-proteins.

The gene encoding the *TEC* tyrosine kinase was identified in murine hepatocarcinoma cells and was later found to be expressed in all murine hematopoietic cell lines examined. Mano, *et al*., *Oncogene, 8:* 417-424 (1993). The other two members of the family, ITK and *BTK,* are selectively expressed at certain stages of T-cell and B-cell development, respectively. Expression of the *ITK* mRNA is induced upon T-cell activation by IL-2 and mutations in *BTK* are thought to be responsible for X-linked agammaglobulinemia (Burton's disease, XLA), a disease characterized by a lack of circulating mature B-cells in affected males. Several different BTK mutations have been described in murine models of XLA, including point mutations in the PH or SH2 domains, which may be involved in functionally important interactions with other proteins.

Cytoplasmic tyrosine kinases have been reported to associate with ligand-activated transmembrane receptors and they appear to initiate or to amplify ligand-induced signals. For example, the T- and B-cell receptors and cytokine receptors in hematopoietic cells associate with certain members of the Src tyrosine kinase family in order to transduce their signals. Upon ligand binding to these receptors, a rapid increase in tyrosine phosphorylation of specific intracellular substrates is observed. These signalling pathways appear therefore to depend on specific intracellular tyrosine kinases recruited and activated by the stimulated receptors. Members of the Src family are expressed in a cell lineage-selective manner, which is consistent with this hypothesis. Several cytokine receptors also interact with and activate JAK family of kinases, which in turn directly phosphorylate transcriptional regulators.

In contrast to cytokine receptors, most growth factor receptors contain intrinsic tyrosine kinase activity. Autophosphorylated tyrosyl residues of some of these receptors, such as platelet-derived growth factor receptor and hepatocyte growth factor/scatter factor receptor bind to SH2 domains of cytoplasmic tyrosine kinases, such as c-Src. The recruitment of a cytoplasmic tyrosine kinase to an activated receptor complex can amplify the signal by associating with other SH2 domain-containing signal transducers and possibly with proteins binding to the SH3 domain.

The present invention provides a new cytoplasmic receptor tyrosine kinase which shares characteristics with members of the *TEC*/*ITK*/*BTK* subfamily and is useful as a marker for cell growth and differentiation and for various types of tumor formation and in the diagnostics and treatment of diseases resulting from deregulated tyrosine phosphorylation. Using a presently-claimed cytoplasmic tyrosine kinase, it is possible to isolate the growth factor or cytokine receptor whose signals are mediated through such kinases by methods standard in the art.

### SUMMARY OF THE INVENTION

The present invention provides novel cytoplasmic tyrosine kinases capable of stimulating growth and/or proliferation of hematopoietic cells. In a preferred embodiment, a protein according to the invention is a *BMX* protein comprising the amino acid sequence shown in SEQ ID NO: 3. Also in a preferred embodiment, the invention provides cDNAs encoding *BMX.*

In a preferred embodiment, a *BMX* tyrosine kinase according to the invention comprises a fragment of the *BMX* protein which is capable of stimulating the growth and/or differentiation of hematopoietic cells, whether in vivo or *in vitro.* Also in a preferred embodiment, the invention provides a DNA encoding a fragment of the *BMX* protein, which fragment is capable of stimulating the growth and/or differentiation of hematopoietic cells.

The present invention also provides an antibody directed against proteins of the invention; including a monoclonal antibody and a hybridoma producing it.

Methods according to the invention comprise means for detecting the growth and/or differentiation of hematopoietic cells comprising the steps of exposing tissue to a detectably-labelled DNA or antibody according to the invention; washing the tissue, and detecting any label which remains in the tissue after washing. Methods for labelling DNA and protein and methods for preparing tissue for hybridization and immunohistochemistry are well-known in the art. The present invention also provides a unique tyrosine kinase, the activity of which is inhibited by specific inhibitors with consequent effects on the growth or differentiation of cells expressing *BMX.*

Additional embodiments and features of the invention will become apparent to the ordinarily-skilled artisan upon consideration of the following detailed description thereof.

### DESCRIPTION OF THE FIGURES

Figure 1 shows amino acid sequences of *BMX* (SEQ ID NO: 3), BTK (SEQ ID NO: 4), ITK (SEQ ID NO: 5), TEC (SEQ ID NO: 6), DSrc28C (SEQ ID NO: 8) and the consensus sequence (SEQ ID NO: 7).
Figure 2A is a Northern blot and hybridization analysis of polyA+ RNA from human umbilical vein endothelial cells (HUVEC) and HT-1080 human fibrosarcoma cells.
Figure 2B is a Western blot of anti-BMX and control anti-SEX immunoprecipitates from HUVEC cells.
Figure 2C shows anti-PTyr Western analysis of *BMX* immunoprecipitates from COS cells transfected with a *BMX*-containing vector or an empty vector (MOCK).
Figure 2D shows SDS-PAGE analysis of immunoprecipitates from COS-transfected cells (BMX),subjected to in vitro kinase reaction.
Figure 3 is a Western blot of *BMX* retrovirus-expressing *BMX* or control virus-infected (c) NIH3T3 cells lysed directly (lanes marked "-") or immunoprecipitated (IP) using anti-*BMX* antiserum.
Figure 4 shows a normal metaphase human chromosomes and an enlargement of the X chromosome showing localization of the *BMX*-encoding gene, along with a schematic depiction of the chromosome.

### DETAILED DESCRIPTION OF THE INVENTION

The present invention provides novel tyrosine kinases, such as the *BMX* tyrosine kinase shown in SEQ ID NO: 3; fragments of said kinases; and DNA encoding said kinases and said fragments. Such cDNAs were isolated from genomic libraries constructed from bone marrow and endothelial cell sources. The *BMX*-encoding cDNA comprises an open reading frame of 2025 bp, encoding 675 amino acids. The protein product comprises a single tyrosine kinase domain, an SH2 domain, and an SH3 domain. The tyrosine kinase domain is approximately 70% homologous to the tyrosine kinase domains of BTK, ITK, and TEC as shown by a comparison of SEQ ID NO: 3 with SEQ ID NO: 4, 5, and 6 respectively. A fragment according to the present invention is any portion of the primary structure of the intact kinase which retains the ability to stimulate or inhibit the growth and/or differentiation of hematopoietic cells.

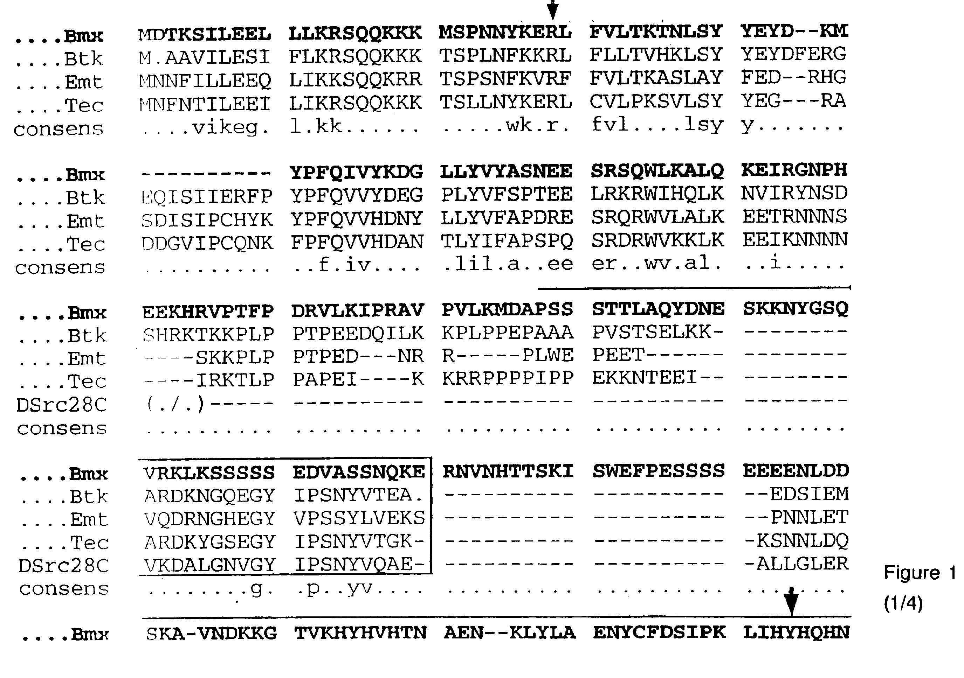

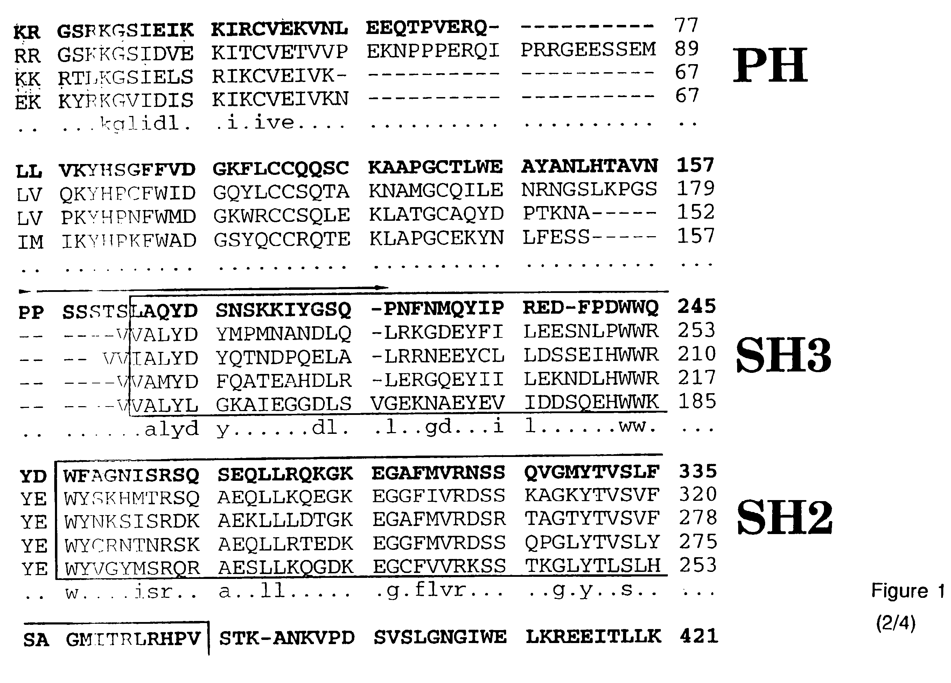

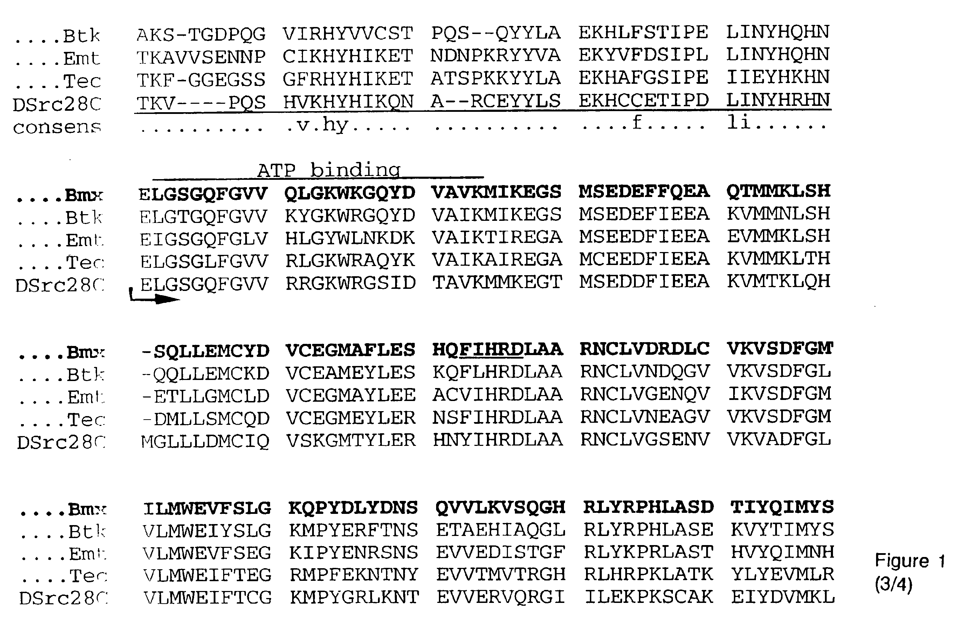

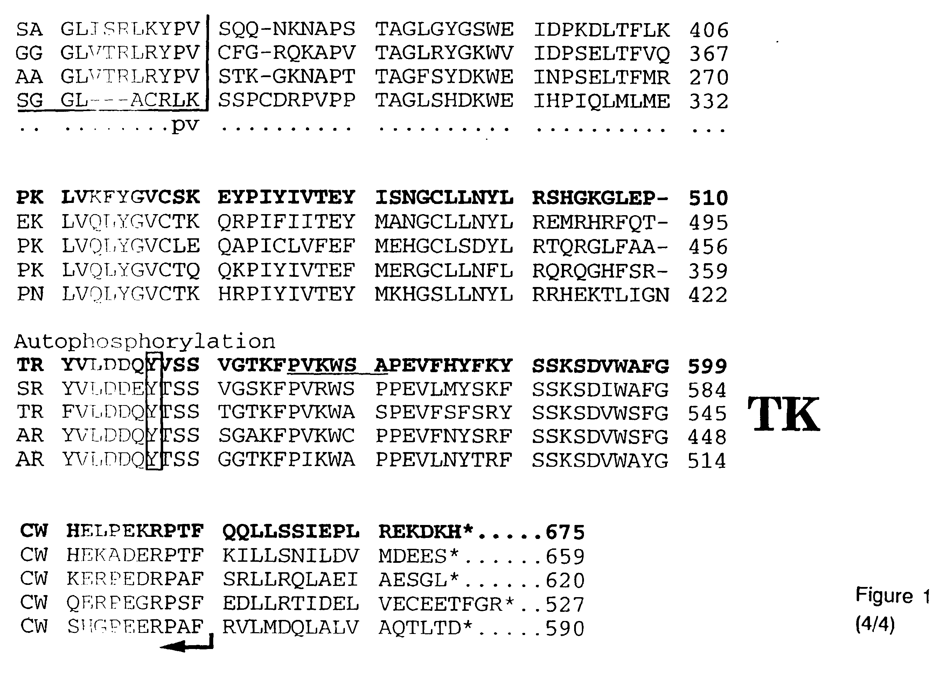

A *BMX* cytoplasmic tyrosine kinase according to the invention has a deduced amino acid sequence, shown in SEQ ID NO: 3, which is closely homologous to the sequences of Btk, Itk, Tec, and *Drosophila melanogaster* Src28C tyrosine kinases. The alignment of the *BMX* sequence with its closest homologous sequences is shown in Figure 1. Inspection of that figure,suggests that the ATG codon at position 36 of the *BMX* cDNA is the translation initiation site. That codon is embedded in a kozak consensus translation initiation sequence (AATATGG).

It has been reported that the variable N-terminal domains of members of the Src family of kinases interact with transmembrane receptors. *Mustelin, et al*., *TIBS: 18:* 215-220 (1993). As shown in Figure 1, the N-terminal region of *BMX* has significant homology with those of *TEC, ITK,* and *BTK.* The N-terminal domain of *BMX* also has a region which compares to the PH (Pleckstrin Homology) consensus sequence found in various GPTase activating (GAP) proteins and in other kinases and cytosketetal proteins. The core of the pleckstrin domain is an antiparallel beta-sheet consisting of seven strands and the C-terminal portion is folded into a long alpha helix. The Pleckstrin domain is electrostatically polarized and contains a putative ligand-binding domain.

In contrast to cytokine receptors, most growth factor receptors contain intrinsic tyrosine kinase activity. Autophosphorylated tyrosyl residues of some growth factor receptors *(e.g.,* platelet-derived growth factor receptor) bind to SH2 domains of cytoplasmic tyrosine kinases, such as c-Src. The recruitment of a cytoplasmic tyrosine kinases to an activated growth factor receptor complex amplifies the signal through that complex by association of the tyrosine kinase with other SH3 and SH2 containing signal transducers.

Claimed *BMX* proteins possess both SH2 and SH3 domains. However, the *BMX* SH3 domain varies from the consensus sequence in that it includes two strongly hydrophilic portions rich in Ser and Glu residues in its C-terminus. This distinction may be due to a splice variation. The foregoing provides substantial evidence that the inventive tyrosine kinases bind active portions of growth factor receptors and, due to the localization of claimed proteins, do so in hematopoietic cell lines.

Accordingly, inventive proteins are useful for stimulating hematopoietic cell lines. Moreover,inventive DNAs are useful as diagnostic reagents in the detection of hematopoietic cell proliferation and oncogenesis. Antibodies and peptides of the invention are additionally useful for inhibiting activity of growth factors. The following examples provide details of the isolation, characterization, localization, and use of inventive proteins, DNAs, and methods for use thereof.

### EXAMPLE 1

### Cloning and Analysis of cDNA Encoding Proteins of the Invention

Total RNA was prepared from normal human bone marrow by guanidium thiocyanate extraction. An aliquot of 2 µg RNA was then reversed-transcribed using, 100 of avian myeloblastosis virus reverse transcriptase in the presence of 0.5 µg oligo-dT primer, 1 µM each of deoxyadenosine triphosphate, deoxyguanine triphosphate, deoxycytosine triphosphate, and deoxythymidine triphosphate, and 10 U RNAsin (Promega, Madison, WI). The reaction buffer contained 50 mM Tris-HC1, pH8.1,6mM MgCl₂, 40 mM KCl and 1mM dithiothreitol. The reaction contents were incubated at 42°C for 1 hour, then at 52°C for 30 minutes, and then at 95°C for 5 minutes.

Approximately 3% of the reverse transcribed cDNA was then amplified by PCR in a reaction volume of 100 ml using 3 U Dynazyme (Finnzymes, Helsinki, FI) in a reaction buffer comprising 1.5 mM MgCl₂ and in the presence of 200 5 µm each of deoxyadenosine triphosphate, deoxycytosine triphosphate, deoxyguanine triphosphate, and deoxythymidine triphosphate. The primers were
5'-GGTCTAGAA(A/g)AA(A/G)TT(C/T)GT(C/G)CAC(A/C)G(G/A) GAC-3'(0.1 5m) (SEQ ID NO: 1) and
5'-GCTCTAGA(G/A)GGCCATCCA(T/C)TT(G/C/A)AC(T/C/A)GG-3 (0.15m) (SEQ ID NO: 2) which represented the sense and antisense primers, respectively. Both primers were obtained from conserved tyrosine kinase domains from known kinases. The protocol for amplification was 90 seconds at 95°C; 120 seconds at 42°C; 180 seconds at 68°C for 35 cycles in a volume of 100 µl in a Perkin Elmer DNA Thermo Cycler 480. A 150 bp cDNA product representing the novel *BMX*-encoding sequences was obtained. That product was subcloned into a pCR vector using a TA-cloning kit (Invitrogen) according to the Manufacturer's instruction.

The PCR-amplified *BMX* product designated B1, described above, was radiolabelled with 32pCTP using, random priming and used tc screen an oligo-dT-primed λgt10 cDNA library constructed from human bone marrow RNA (Clontech). The B1 cDNA included the sequence obtained by PCR amplification and flanking open reading frame, predicting a cytoplasmic tyrosine kinase very closely related to the newly identified Btk kinase. When the B1 cDNA was used to probe Northern blots, no specific signal was detected in any cell lines examined. Nevertheless, according to results obtained by reverse transcription-PCR amplification of RNA, *BMX* appeared to be expressed, not only in bone marrow, but also in endothelial cells. Therefore, a human cDNA library derived from endothelial cell RNA was screened in order to obtain full length cDNA. Several positive plaques were chosen and the longest *BMX* cDNA insert of approximately 2.4 kb (E7) was subcloned into a pGEM plasmid (Promega) and sequenced on both strands using the dideoxy chain termination method of Sanger. The E7 clone was deposited with the American Type Culture Collection, 12301 Parklawn Drive, Rockville, MD 20852 on October 5, 1994 as accession No. ATCC 75907 . Computer analyses of the sequences were performed using the GCG program as reported in Devereux, *et al*., *Nucl. Acids Res., 12:* 387-395 (1984), incorporated by reference herein. The *BMX* sequence obtained is shown in Figure 1 and in SEQ ID NO: 3.

The E7 subclone contained an open reading frame capable of encoding 675 amino acids. The deduced amino acid sequence was closely homologous to the sequences of TEC, ITK, and BTK and to the *Drosophila melanogaster* Src28C tyrosine kinase. Sequence alignment suggested that the ATG at position 36 of the cDNA was the translation initiation site.

The *BMX* protein has an N-terminal region of 210 residues comprising a PH domain (shaded region in Fig. 1), but no consensus myristylation site. The variable amino terminal domains of the Src family are involved in interaction with transmembrane receptors. Accordingly, it is interesting to note that the long N-terminal region of *BMX* shares a high degree of homology with the *Btk, Itk* and *Tec* kinases. This part of the molecule may have a role in associating with as yet unknown receptors or signal transducers. In this region, the sequences of these four tyrosine kinases are rich in basic amino acid residues and they fit the consensus for the PH domain found in a number of proteins, such as certain GTPase activating proteins (GAP) and GDP-GTP exchange factors (such as SOS1), in kinases such as sARK and RAC, and in dynamin, kinesin and spectrin.

The PH domain is followed by an SH3 and an SH2 domain (boxed region in Figure 1). For comparison, the corresponding regions of *Drosophila melanogaster* Src28C TK sequence are also shown in Figure 1. The sequences between amino acid residues 185-206 and 207-228 (horizontal arrows in Figure 1) of *BMX* are about 80% identical with each other both at the nucleotide and at the amino acid level, suggesting that this stretch originated from a duplication of the *BMX* DNA sequence. The latter stretch (207-228) belongs to the N-terminal portion of the *BMX* SH3 domain.

Although features of SH3 domains are common to all members of the *BMX*/*BTK*/*ITK*/*TEC* tyrosine kinase family, some of the sequences diverge from the consensus. The C-terminal portion of the *BMX* SH3 sequence (downstream of the WW motif at position 243) diverges from those of the other kinases, and contains two strongly hydrophilic stretches, rich in Ser and Glu residues. In contrast, the SH2 domain is well-conserved in the *BMX* sequence when compared with the other tyrosine kinases (Fig. 1). Within the tyrosine kinase domain (arrows) the ATP binding motif containing the Gly(434)XGlyXXGly sequence and the Lys435 residue are marked in Fig 1. The Tyr566 residue of *BMX* corresponds to the conserved Tyr416 autophosphorylation site of c-Src. The catalytic domain is followed by a short C-terminal tail, where nonconserved autophosphorylation sites are found. Multiple stop codons were found after codon 675 of the *BMX* sequence.

Polyadenylated RNAs from several human fetal and adult tissues were analyzed for *BMX* RNA by Northern blotting and hybridization. *BMX* transcripts were prominent in both fetal and adult heart. Weaker signals were obtained from fetal lung and kidney; and from adult skeletal muscle, placenta, lung, liver, testis, ovary, and small and large intestine. Adult kidney, pancreas and prostate gave signals only after a very long exposure of the autoradiogram, whereas no signal could be obtained from fetal or adult brain or fetal liver or kidney. Thus, *BMX* appears to be more widely expressed than the related Btk, Itk and Tec tyrosine kinases. At least part of these hybridization signals may be derived from hematopoietic cells in these organs.

### EXAMPLE 2

### Expression and Analysis of the BMX Protein

A *BMX* retrovirus was constructed into pBABEpuro vector. The pBABEpuro vector is reported in Morgenstern, *et al., Nucl. Acids Res., 12:* 387-395 (1990). The resulting vector was then transfected into BOSC23 cells which as reported in Pear, *et al., Proc. Natl. Acad. Sci. (USA), 90:* 8392-8396 (1993), incorporated by reference herein. The supernatant was then collected 48 hours later and used to infect NIH3T3 cells. The infected cells were selected by growth for 2 weeks in puromycin. COS cells were transfected with 10 µg of the pMT2 vector, which is reported in Kaufman, *et al, Cell. Biol., 9*: 946-958, incorporated by reference herein, containing the *BMX* cDNA insert using the calcium phosphate method. After 36-48 hours of growth, the cells were extracted with the electrophoresis sample buffer 2.5 % sodium dodecyl sulfate, 0.125 M Tris-HCl, pH 6.8 for Western blotting or with ice-cold RIPA buffer (50mM TrisHCl pH 7.5, NaCl 150 mM, 1% Triton X100, 1% sodium deoxycholate, 0.1% SDS, containing 10 mg/ml pepstatin, 100 5g/ml leupeptin, 0.05 TIU/ml aprotinin, 1 mM PMSF and 1 mM activated sodium orthovanadate) for immunoprecipitation. The clarified supernatants were immunoprecipitated with 5 µl of anti-*BMX* antiserum raised in rabbits using a GST-fusion protein (Pharmacia) engineered to express *BMX* amino acid residues 599-675. Preimmune serum and anti-SEX antiserum against an unrelated protein (L.T., in preraration) were used as controls.

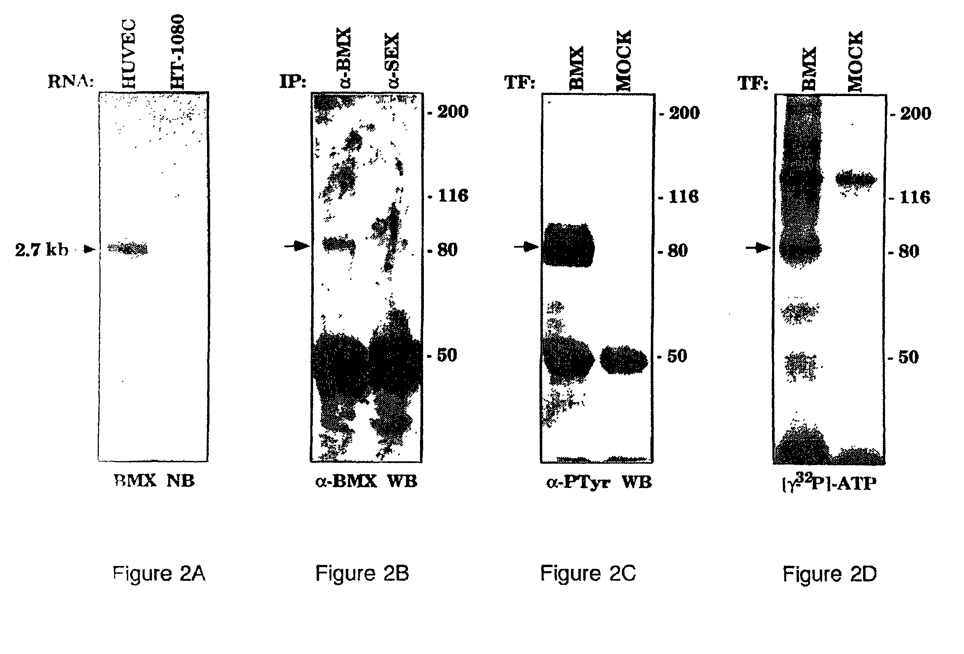

Samples were analyzed in 7.5% SDS-PAGE followed by Western blotting and detection using a 1: 1000 dilution of the anti-*BMX* antiserum (Figure 2B) or the PY20 anti-phosphotyrosine monoclonal antibodies as shown in Figure 2C (Zymed), followed by peroxidase-conjugated antibodies against mouse immunoglobulins and ECL detection according to the manufacturer's instructions (Amersham). Alternatively, the immunoprecipitates were subjected to a kinase reaction in 25 mM HEPES, pH 7.2, 100mM NaCl, 5 mM MgCl₂, 10 mM MnCl₂ and 10 mCi [³²P]-ATP for 10 minutes at room temperature, followed by SDS-PAGE and autoradiography.

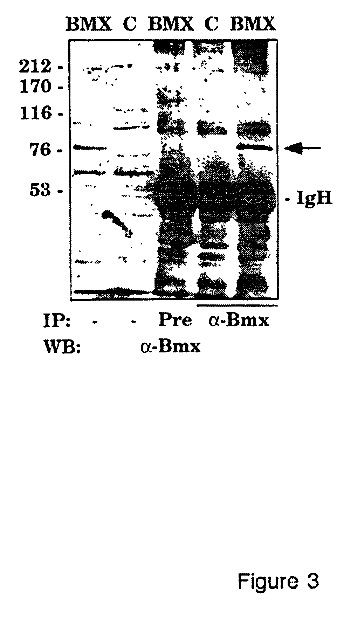

Results are shown in Figures 2A-2D. Figure 2A shows Northern blotting analysis of RNA from human umbilical vein endothelial cells using the *BMX* probe (labelled *BMX* NB in the Figure). A 2.7 kb mRNA band is seen. Immunoprecipitation of the *BMX* protein followed by immunoblotting with anti-*BMX* antibodies resulted in the detection of a weak band of 80 kD apparent molecular weight as shown in Figure 2B and 3. That band was not seen in control immunoprecipitates. Immunoprecipitation and immunoblotting of *BMX* from NIH3T3 cells expressing a *BMX* retrovirus and from COS cells transfected with a *BMX* plasmid expression vector also resulted in the detection of a 80 kD polypeptide, which was tyrosyl phosphorylated as shown in Figure 2C (α-PTyr WB). However, that polypeptide was only weakly labelled in immunocomplex kinase reactions [³²P]-ATP, as shown in Figure 2D.

### EXAMPLE 3

### Chromosomal Localization of the BMX Gene

A Southern blot made from 24 interspecies somatic cell hybrids was obtained from the Mutant Cell Repository of the Coriell Institute (Camden, NJ) .

In order to determine the chromosomal localization of the *BMX* gene, DNAs from human rodent somatic cell hybrids containing defined sets of human chromosomes were analyzed by Southern blotting and hybridization with the *BMX* probe. Among 24 DNA samples, human-specific signals were observed in two human/Chinese hamster hybrids, one containing human chromosomes 1 and X and the other only the human X chromosome. This analysis indicated that the *BMX* gene is located in chromosome X. Thus the name Bone Marrow kinase gene on the X chromosome, *BMX,* was chosen. A human placenta cosmid library in pWE15, described in Lichter, *et al*., *Human Genet., 80:* 224-234 (1988), and a human X-chromosome yeast artificial chromosome (YAC) library were screened with the [³²P]-labelled insert of the *BMX* cDNA. Positive clones were rescreened until pure, and verified by Southern blotting and hybridization.

Slides with human metaphase chromosomes, prepared from 5-bromo deoxyuridine-synchronized lymphocyte cultures were prehybridized and hybridized essentially as described in Lichter, et *al., Human Genet., 80:* 224-234 (1988), incorporated by reference herein. Four *Eco*RI fragments (1, 1.5, 2.3 and 2.5 kb) of the *BMX* cosmid were used as probes after labeling with biotin-16-dUTP by nick translation. The four probes were pooled in a mixture of 50% formamide, 2xSSC, 1% Tween 20, 10% dextran sulfate, 25µg Cot-I DNA and 8 mg salmon sperm DNA, denatured at 75 °C fcr minutes and then preannealed at 37 °C for 30 minutes. After hybridization, the slides were stringently washed, the signal was made fluorescent, amplified, and the chromosomes were counterstained with propidiumiodide and DAPI. The results were analyzed and photographed in a confocal laser scanning microscope (Zeiss).

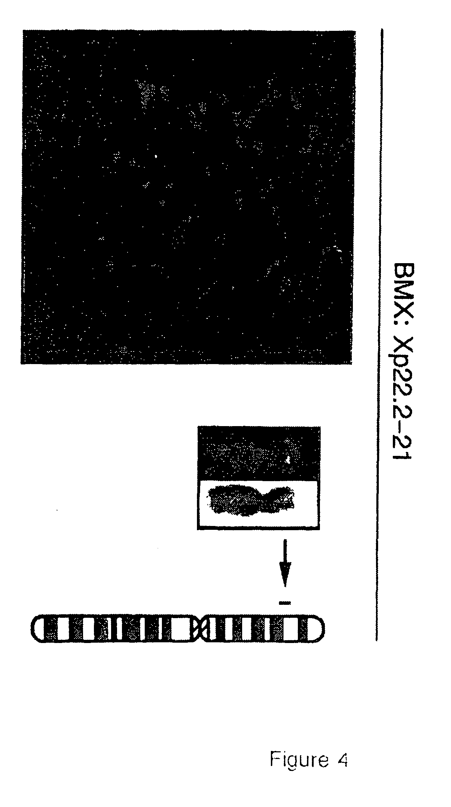

A genomic cosmid clone isolated by hybridization with the *BMX* cDNA gave signals from chromosomes X and 18 in the hybrid panel. Therefore, all four *BMX*-positive *Eco*RI fragments of this clone were labelled and used inflorescence in situ hybridization to further localize the *BMX* gene. This probe gave a specific signal in Xp22.2-p21 (Fig. 4). The *BMX* cDNA was then hybridized to YACs from the Xp21 and Xp22 region. The 400 kb ICRF YAC 900G1096 and the 350 kb CEPH YAC244G7 were positive for *BMX*. Both of these YACs were non-chimeric as analyzed by FISH; the former was positive for the DXS207 and DXS197 loci and the latter for only DXS197. As *BMX* was negative on YACs positive for DXS197 and DXS43, this maps *BMX* between the DXS197 and DXS207 loci in band Xp22.2.

The invention has been described in terms of its preferred embodiments. Accordingly, additional aspects of the invention will be apparent to the ordinarily-skilled artisan upon reading the present application.

## Claims

1. A protein having tyrosine kinase activity comprising the amino acid sequence shown in SEQ ID NO:3 or a part of said sequence having tyrosine kinase activity capable of stimulating growth of hematopoietic cells.

2. The protein according to claim 1 which is capable of stimulating growth of hematopoietic cells.

3. A recombinant DNA encoding the protein according to claim 1.

4. A method for detecting growth of hematopoietic cells, comprising the steps of
(a) exposing tissue comprising said hematopoietic cells to a detectably labelled DNA according to claim 3;
(b) washing said tissue; and
(c) detecting said label in said tissue.

5. An antibody which is specifically reactive with the protein according to claim 1 and which do not recognise other related tyrosine kinases.

## Patentansprüche

1. Protein mit Tyrosinkinase-Aktivität, umfassend die in SEQ ID NO: 3 dargestellte Aminosäuresequenz oder einen Teil dieser Sequenz, der Tyrosinkinase-Aktivität hat und in der Lage ist, das Wachstum von hämatopoetischen Zellen zu stimulieren.

2. Protein nach Anspruch 1, das in der Lage ist, das Wachstum von hämatopoetischen Zellen zu stimulieren.

3. Rekombinante DNA, die das Protein von Anspruch 1 codiert.

4. Verfahren zum Nachweis des Wachstums von hämatopoetischen Zellen, umfassend die Schritte
(a) Inkontaktbringen von Gewebe, umfassend die hämatopoetischen Zellen, mit einer nachweisbar markierten DNA nach Anspruch 3;
(b) Waschen des Gewebes; und
(c) Nachweisen der Markierung in dem Gewebe.

5. Antikörper, der spezifisch mit dem Protein nach Anspruch 1 reagiert und der andere verwandte Tyrosinkinasen nicht erkennt.

## Revendications

1. Protéine ayant une activité tyrosine kinase comprenant la séquence d'acides aminés présentée dans SEQ ID NO:3 ou une partie de ladite séquence ayant une activité tyrosine kinase capable de stimuler la croissance de cellules hématopoïétiques.

2. Protéine selon la revendication 1 qui est capable de stimuler la croissance de cellules hématopoïétiques.

3. ADN recombiné codant la protéine selon la revendication 1.

4. Procédé de détection de la croissance de cellules hématopoïétiques, comprenant les étapes :
(a) d'exposition de tissu comprenant lesdites cellules hématopoïétiques à un ADN marqué de manière décelable selon la revendication 3 ;
(b) de lavage dudit tissu ; et
(c) de détection dudit marqueur dans ledit tissu.

5. Anticorps qui est spécifiquement réactif à la protéine selon la revendication 1 et qui ne reconnaît pas d'autres tyrosine kinases apparentées.
